# EUROPÄISCHE PATENTSCHRIFT

(11) **EP 2 988 706 B1**
(45) Veröffentlichungstag und Bekanntmachung des Hinweises auf die Patenterteilung: **05.04.2017**
(21) Anmeldenummer: 14739666.7
(22) Anmeldetag: 17.04.2014
(51) Int. Cl.: A61F 2/915, A61F 2/848

(54) **STENT**
STENT
STENT

(30) Priorität: 22.04.2013 DE 102013104062
(43) Veröffentlichungstag der Anmeldung: 02.03.2016
(73) Patentinhaber: Novatech SA, 13705 La Ciotat Cedex (FR)
(72) Erfinder: NISSL, Thomas, 37318 Mackenrode (DE)
(74) Vertreter: Griepenstroh, Jörg
(86) Internationale Anmeldenummer: PCT/EP2014/001033
(87) Internationale Veröffentlichungsnummer: WO 2014/173511

(56) Entgegenhaltungen:
- WO-A2-2012/071542
- GB-A- 2 494 820
- US-A1- 2003 139 799

## Beschreibung

Die Erfindung betrifft einen Stent mit den Merkmalen im Oberbegriff des Patentanspruchs 1.

Ein Stent ist ein medizinisches Implantat, das in ein Hohlorgan eingebracht wird, um es offen zu halten. Es handelt sich um eine radial ausdehnbare Endoprothese, die in der Zielposition radial vergrößert wird. Stents können aus selbstausdehnenden Werkstoffen bestehen oder auch durch von innen aufgebrachte Radialkräfte ausgedehnt werden, beispielsweise durch eine Ballondilatation. Stents sind aus unterschiedlichen Materialien gefertigt. Zumeist bestehen sie aus Metallen, wie rostfreiem Stahl, oder auch aus Formgedächtnislegierungen, wie beispielsweise Nitinol.

Bei Stents aus Metallen zählt eine Bauform zum Stand der Technik, bei welcher der im Allgemeinen rohrförmige Körper des Stents mehrere, serpentinenartige, ringförmig ausdehnbare Elemente aufweist. Diese serpentinenartigen Elemente besitzen Bögen, die in Axialrichtung des Stents weisen. Die Bögen sind über Schenkel verbunden, die in Axialrichtung weisen. In Axialrichtung aufeinander folgende, serpentinenartige, ringförmig ausdehnbare Elemente sind dabei über so genannte Konnektoren miteinander verbunden, die in der Regel an den Enden der benachbarten Elemente angeordnet sind. Eine solche Anordnung ist aus der WO 2006/022949 A1 bekannt. Die Konnektoren sind so konfiguriert, dass eine Expansion der serpentinenartigen, ringförmigen Elemente möglich ist. Gleichzeitig soll der Stent aber während der transluminalen Implantation einen möglichst geringen Radius haben. In der WO 2006/022949 wird daher vorgeschlagen, dass sich die Konnektoren in der komprimierten Stellung überlagern.

Die DE 29 825 178 U1 sieht spiralförmige Konnektoren mit mehreren Armen vor, die zu den Bögen der benachbarten Elemente führen. Die spiralförmigen Konnektoren werden bei Expandieren des Stents gewissermaßen abgewickelt, so dass sich die serpentinenartig, ringförmig ausdehnbaren Elemente, welche die Stützfunktion des Stents übernehmen, in Radialausrichtung ausdehnen können.

Die GB 2 494 820 A1 offenbart einen Stent zur Überbrückung eines Aneurysmas. Im mittleren Bereich soll sich der Stent leicht aufweiten lassen, um Okklusionsmaterial in das benachbarte Aneurysma einzubringen. Der Stent besitzt zwischen serpentinenartigen Elementen Konnektoren, die sich in Umfangsrichtung des Stents erstrecken und eine Krümmung in Umfangsrichtung des Stents besitzen. Die Krümmung in Umfangsrichtung ist bei einem expandierten Stent so groß, dass die Konnektoren nach radial außen über den Umfang der serpentinenartigen Elemente vorstehen. Während die serpentinenartigen Elemente im Wesentlichen einen zylindrischen Bereich beschreiben, ergibt sich im mittleren Abschnitt im Bereich der Konnektoren eine nach außen gerichtete Wölbung zwischen den einzelnen Konnektoren. Hier ist der Abstand zwischen den Konnektoren größer als im Bereich der serpentinenartigen Elemente, damit Okklusionsmaterial durch diesen Bereich in das angrenzende Aneurysma eingebracht werden kann. Eine Verengung der Abstände zwischen den Streben wäre beim Einbringen von Okklusionsmaterial in das Aneurysma hinderlich.

Zum Stand der Technik ist ferner die WO 03/061528 A1 zu nennen, die einen sogenannten Multi-Layer-Stent betrifft. Es gibt serpentinenartige Segmente und Konnektoren. Die Konnektoren können stark gebogen sein und über den zylindrischen Bereich des Stents nach außen vorstehen.

Die DE 200 23 387 U1 offenbart einen Stent mit serpentinenartigen Elementen und dazwischen angeordneten Konnektoren. Die Konnektoren sind als gerade Stege ausgeführt.

Die Konnektoren können aber auch gekrümmt sein, wie beispielsweise aus der US 2007/0208416 A1 hervorgeht. Die Konnektoren können eine Längenveränderung des Stents zulassen.

Die aus der WO 2012/071542 A2 bekannten Konnektoren können bei einigen Ausführungsbeispielen Y-förmig konfiguriert sein. Ein Basisarm ist mit einem Bogen am Ende eines ersten serpentinenartigen Elements verbunden. Die Gabelarme sind Y-förmig, teilen sich aber nochmals auf, so dass sie jeweils mit drei benachbarten Bögen am Ende des anderen serpentinenartigen, ringförmig aus den Barrenelementen verbunden sind.

Die EP 1 703 859 B1 offenbart einen Stent zur Ablage auf einer Biegung eines Körperhohlraumes. Der Stent besitzt serpentinenartige Elemente und dazwischen angeordnete Konnektoren die gerade oder gekrümmt sein können. Die Konnektoren sind nicht gabel- oder Y-förmig ausgebildet. Im Unterschied hierzu wird in der DE 198 22 157 A1 ein radial aufweitbarer Stent zur Implantierung in einem Körpergefäß vorgeschlagen, bei welchem sich gabelnde Konnektoren vorgesehen sind, die allerdings noch einmal in sich verschlungen sind. Dadurch sollen zusätzliche Aufdehnungsmöglichkeiten geschaffen werden um einen zweiten, nicht aufgeweiteten Stent durch die radialen Öffnungen hindurchzuführen oder sogar im Bereich der Öffnungen radial aufzuweiten. Dabei muss die Querschnittsfläche der Öffnungen in der Mantelfläche des Stents stark vergrößert werden. Der Stent ist beispielsweise zur Verlegung im Bereich von Gefäßverzweigungen vorgesehen.

Der Erfindung liegt die Aufgabe zugrunde, einen Stent in Form eines im Allgemeinen rohrförmigen Körpers mit mehreren serpentinenartigen, ringförmig ausdehnbaren Elementen dahingehend zu verbessern, dass er nach dem radialen Ausdehnen der serpentinenartigen Elemente besser gegen axiale Verlagerung innerhalb des Körpergefäßes gesichert ist.

Diese Aufgabe ist bei einem Stent mit den Merkmalen des Patentanspruchs 1 gelöst.

Vorteilhafte Weiterbildungen der Erfindung sind Gegenstand der Unteransprüche.

Ein erfindungsgemäßer Stent in Form eines im Allgemeinen rohrförmigen Körpers mit mehreren serpentinenartigen, ringförmig ausdehnbaren Elementen besitzt in bekannter Weise Konnektoren. Die Konnektoren sind an den Bögen befestigt, die sich in Axialrichtung des Stents an den ersten und zweiten Enden eines jeweiligen serpentinenartigen, ringförmig ausdehnbaren Elements befinden. Die Konnektoren, die sich im nicht expandierten Zustand des Stents in Umfangsrichtung des Stents erstrecken, besitzen eine Krümmung in Umfangsrichtung, wobei die Krümmung im expandierten Zustand des Stents in Umfangrichtung so groß ist, dass die Konnektoren nach radial außen über den Umfang der serpentinenartigen Elemente vorstehen. Dadurch bilden die Konnektoren im expandierten Zustand des Stents ringförmige, nach außen vorstehende Verdickungen, die einer Verlagerung des Stents in Längsrichtung der Körperhöhle entgegenwirken.

Im nicht expandierten Zustand soll der Stent einen möglichst kleinen Außendurchmesser besitzen, um ihn minimalinvasiv platzieren zu können. Der Radius des Stents ist der Kehrwert der Krümmung. Diese Krümmung der Konnektoren bleibt bei der Expansion des Stents im Wesentlichen erhalten. Da bei der radialen Expansion des Stents die Krümmung der übrigen Mantelfläche abnimmt, stehen die stärker gekrümmten Konnektoren nach radial außen vor. Sie werden aus der Mantelfläche der serpentinenartigen Elemente heraus verlagert.

Wenn die Konnektoren hinreichend biegesteif sind, bleibt die Krümmung theoretisch vollständig erhalten. In der Praxis nimmt die Krümmung jedoch ein klein wenig ab, da beim Expandieren des Stents entgegengesetzt wirkende Drehmomente an den Enden der Konnektoren angreifen. Die Krümmung bleibt in diesem Fall nur im Wesentlichen erhalten, da sich die Verformung der radial vorstehenden Bereiche im elastischen Verformungsbereich des Werkstoffes bewegt.

Die Konnektoren erstrecken sich in der Ausgangsposition über ein Drittel bis zwei Drittel des Umfangs des nicht expandierten Stents, d.h. über 120° bis 240°. Dieser Winkel, bezogen auf den Umfang des Stents, wird bei dessen Expansion kleiner, da die Konnektoren bei Expansion des Stents nicht länger werden. Im expandierten Zustand erstrecken sich über einen Umfangsbereich von 30° bis 85°. Die von den Konnektoren überspannten Umfangsbereiche überlappen sich, so dass die Konnektoren einen radial nach außen vorstehenden Kranz bilden.

Die sich an die Konnektoren anschließenden, serpentinenartigen Elemente werden beim Expandieren gegensätzlich zueinander verdreht. Das ist darauf zurückzuführen, dass sich die Konnektoren nicht längen aber gleichzeitig nach außen ausgestellt werden, so dass sich die Konnektoren nicht wie in der Ausgangslagen um z.B. 120° eines kleinen Zylindermantels mit einem Durchmesser von z.B. 5 mm erstrecken, sondern über z.B. 30° eines größeren Zylindermantels mit einem Durchmesser von 20 mm.

Die Konnektoren erstrecken sich gewissermaßen schraubenlinienförmig wie ein vielgängiges Gewinde zwischen den benachbarten, serpentinenartigen Elementen. Die Orientierung aller in Axialrichtung des Stents aufeinander folgender Konnektoren kann identisch sein. Es wird jedoch als vorteilhaft angesehen, wenn die Konnektoren an einem axialen Ende eines serpentinenartigen Elements gegensinnig zu den Konnektoren an dem anderen axialen Ende des serpentinenartigen Elements verlaufen. Dadurch können benachbarte serpentinenartige Elemente abwechselnd gegensinnig verdreht werden, ohne dass der gesamte Stent gedreht wird. Ein Beispiel: Bei einer Anordnung E-K1-E-K2-E, wobei in dieser Folge E für serpentinenartiges Element steht und K1 und K2 für gegensinnig umlaufende Konnektoren stehen, können die endseitigen Elemente E ihre Ausgangslage beibehalten und nur das mittlere Element E wird in Umfangsrichtung gegensinnig zu den endseitigen Elementen E gedreht. Die Konnektoren K1 und K2 werden dabei in Radialrichtung aufgespreizt. Bei gleichsinnig orientierten Konnektoren, also bei einer Anordnung E-K1-E-K1-E müssten die äußeren Elemente E in dieselbe Richtung aber um den doppelten Betrag gedreht werden.

Die Konnektoren können mit den einander zugewandten Enden der serpentinenartigen Elemente verbunden sein. Das heißt, dass die Konnektoren mit Außenseiten der Bögen der serpentinenartigen Elemente verbunden sind.

Alternativ ist es möglich, dass die Konnektoren mit den voneinander abgewandten Enden der serpentinenartigen Elemente verbunden sind, also an eine Innenseite eines Bogens anschließen. Die Konnektoren erstrecken sich bis in den von den serpentinenartigen Elementen überdeckten Bereich. Dadurch sind sie etwas weicher aufgehängt, weil es bis zu dem Befestigungspunkt an der Innenseite eines entfernt liegenden Bogens einen längeren Hebelarm gibt.

Ebenso sind Mischformen möglich, bei denen der Konnektoren einerseits mit einer Außenseite eines Bogens und andererseits mit einer Innenseite eines Bogens verbunden ist. Dass die Konnektoren mit einem Ende des serpentinenartigen Elements verbunden sind, bedeutet im Kontext dieser Erfindung nicht zwangsläufig, dass es sich um das nächstliegende Ende des serpentinenartigen Elements handelt. Es kann sich auch um das andere, entfernt liegende Ende des serpentinenartigen Elements handeln, also um eine Verbindung mit der Innenseite des betreffenden Bogens.

Die Konnektoren besitzen drei Arme und sind also Y-förmig konfiguriert. Das heißt, dass ein Basisarm des Konnektors mit einem Bogen am Ende des ersten serpentinenartigen, ringförmig ausdehnbaren Elements verbunden ist, während die beiden Gabelarme des Y-förmigen Konnektors jeweils mit einem von zwei benachbarten Bögen am Ende des anderen serpentinenartigen, ringförmig ausdehnbaren Elements verbunden sind.

Die Y-förmigen Konnektoren, die auch als geteilte Konnektoren bezeichnet werden können, werden durch die die Radialkraft der einzelnen serpentinenartigen Elemente unterstützt. Gleichzeitig bleibt die Flexibilität des Stents gewährleistet. Die Konnektoren können zudem in der komprimierten Ausgangslage, das heißt bei einem Durchmesser des Stents von zum Beispiel 5 mm nebeneinander liegen. Die Konnektoren überlappen sich nicht in Radialrichtung des Stents, so dass es im Bereich der Konnektoren zu keiner Verdickung kommt. Natürlich kommt es ebenso wenig zu einer Überlagerung, wenn der Stent seinen Enddurchmesser erreicht. Ein solcher Enddurchmesser kann beispielsweise 20 mm betragen. Es handelt sich zum Beispiel um einen Luftröhrenstent.

Darüber hinaus haben die Y-förmigen Konnektoren die besondere Eigenschaft, dass beim Spreizen der Gabelarme, wie es beim Dehnen des Stents der Fall ist, die Gabelarme eine Kraft auf den Gabelpunkt des Konnektors ausüben. Der Gabelpunkt ist der Punkt, an dem die Gabelarme mit dem Basisarm verbunden sind. Der Gabelpunkt wird gegenüber dem von den benachbarten Elementen aufgespannten Zylindermantel nach radial außen verlagert. Das bedeutet, dass die Konnektoren in der expandierten Position radial weiter nach außen über den im Wesentlichen zylindrischen Stent vorstehen als die serpentinenartigen Elemente. Die Konnektoren üben eine nach außen gerichtete Radialkraft auf das umgebende Gewebe aus. Diese Radialkraft bzw. die nach radial außen vorstehenden Konnektoren bewirken eine bessere Verankerung des Stents in der Körperhöhle und sichern den Stent zusätzlich mechanisch gegen Längsverschiebungen innerhalb der Körperhöhle.

Erfindungsgemäß wird es daher als besonders vorteilhaft angesehen, wenn mehrere der ausdehnbaren, serpentinenartigen Elemente Konnektoren in Axialrichtung des Stents aufeinander folgen, so dass sich eine Folge von Konnektoren und serpentinenartigen Elementen ergibt, die sich funktional im Hinblick auf die nach außen gerichtete Radialkraft ergänzen, weil jedes serpentinenartige Element an wenigstens einem Ende von einem Konnektor unterstützt wird. Die Konnektoren wirken nicht nur als Verbindungsglied zwischen den serpentinenartigen Elementen, sondern als Verankerungselement für den Stent. Je mehr Konnektoren vorgesehen sind, desto besser ist auch die Verankerung des Stents in der Körperhöhle. Je nach Länge des Stents können 3 bis 5 oder auch mehr Konnektoren zwischen einer entsprechenden Anzahl in Axialrichtung aufeinander folgenden Elementen angeordnet sein. Der expandierte Stent ähnelt dabei von außen betrachtet einem Bambusrohr. Die beim Bambus knotenförmigen Verdickungen entsprechen den nach radial außen weisenden Konnektoren, während die serpentinenartigen, ringförmig ausdehnbaren Elemente den zylindrischen Abschnitten dazwischen entsprechen. Diese Geometrie besteht natürlich nur in der expandierten Position.

Sollte es erforderlich sein, den Stent wieder zu entfernen, wobei der Stent wieder komprimiert wird, um ihn auf einen Ausgangsdurchmesser zurückzuführen, werden die Konnektoren wieder in ihrer Ausgangsposition zurückverlagert und stehen nicht mehr in Radialrichtung über die serpentinenartigen Elemente vor. Das erleichtert letztendlich auch die Entnahme des Stents.

Um eine möglichst gleichmäßige Verankerung durch die nach außen wirkenden Konnektoren zu erreichen, ist bevorzugt vorgesehen, dass alle Bögen eines serpentinenartigen Elements über die Konnektoren mit den Bögen eines benachbarten serpentinenartigen Elements verbunden sind. Grundsätzlich können die serpentinenartigen Elemente auch miteinander gekoppelt werden, wenn jeder zweite, dritte oder vierte Bogen mit einem Konnektor versehen ist. Das serpentinenartige Element kann daher auch Bögen ohne angeschlossene Konnektoren besitzen. Da die Konnektoren jedoch nicht nur die Funktion haben, die serpentinenartigen Elemente miteinander zu verbinden, sondern aktiv dazu beizutragen, den Stent zu verankern, wird ein Maximum an Konnektoren angestrebt.

In bevorzugter Ausführungsform ist der Basisarm des Y-förmigen Konnektors in einer Abwicklungsebene des Stents betrachtet gekrümmt. Dadurch befindet sich der mit dem Basisarm verbundene Bogen des einen Elements in einem anderen Umfangsabschnitt als diejenigen Bögen des anderen Elements, mit welchen die Gabelarme verbunden sind. Die Krümmung des Basisarms verläuft also innerhalb der zylindrischen Mantelfläche des Stents. Würde man den Stent als Abwicklung eines Zylindermantels betrachten, liegt die Krümmung in der Abwicklungsebene, welche der Mantelebene des Stents entspricht.

Die Tatsache, dass der Verbindungspunkt des Basisarms in einem anderen Umfangsabschnitt liegt als der Gabelpunkt, über den sich die Gabelarme anschließen, führt dazu, dass der Gabelpunkt beim Aufweiten des Stents aus der Umfangsebene nach radial außen verlagert werden kann. Der Basisarm dient dabei unterstützend als Schwenkarm und stützt den Gabelpunkt, so dass dieser in der ausgeschwenkten Position verbleibt.

In vorteilhafter Weiterbildung sind auch die Gabelarme in einer Abwicklungsebene des Stents betrachtet gekrümmt, so dass der Gabelpunkt, an dem der Basisarm mit den beiden Gabelarmen verbunden ist, in einem anderen Umfangsabschnitt des Stents liegt als diejenigen Bögen der ringförmig ausdehnbaren Elemente, an denen die Gabelarme befestigt sind.

Die Krümmung der Gabelarme hat dieselbe Funktion wie die Krümmung der Basisarme: Der Gabelpunkt soll bei seiner Verlagerung nach radial außen besser gestützt und geführt werden. Der notwendige Freiheitsgrad des Gabelpunktes wird dadurch erreicht, dass die Enden, an denen die Gabelarme befestigt sind, relativ zueinander verlagert werden. Die Krümmung der Gabelarme unterstützt dabei ein Verschwenken der Gabelarme relativ zueinander und damit auch des gemeinsamen Gabelpunktes. Der nach außen ausgestellte Gabelpunkt wird also in der expandierten Position des Stents durch drei Arme gestützt, einerseits den Basisarm und andererseits durch die beiden Gabelarme. Diese 3-Punkt-Abstützung ermöglicht eine eindeutige Kraftverteilung auf die drei Arme des Konnektors. Die Lage des Gabelpunkts ist aufgrund der Geometrie der Gabelarme exakt definiert. Nur durch Veränderung des gegenseitigen Abstands der Bögen beim Zusammendrücken oder Expandieren des Stents kann die Position des Gabelpunktes verlagert werden. Ohne diese Verlagerung bleibt er in der expandierten Position des Stent sicher und zuverlässig bei der Ausstellung des Gabelpunkts, so dass auch langfristig die Fixierung des Stents über die nach außen gerichteten Konnektoren gewährleistet ist.

Versuche haben gezeigt, dass es besonders vorteilhaft ist, wenn die Gabelarme, die zur Krümmung jeweils einen Gabelarmbogen besitzen, in einer Abwicklungsebene des Stents betrachtet jeweils unterschiedlich gekrümmt sind. Die Gabelarmbögen besitzen mithin voneinander abweichende Krümmungsradien. Diese Abweichungen in den Krümmungsradien führen dazu, dass die im Gabelpunkt miteinander verbundenen Gabelarme beim Auseinanderbewegen zweier benachbarter Bögen voneinander abweichende Bewegungen ausführen müssen. Diese Bewegung trägt je nach Krümmung der Gabelarmbögen dazu bei, dass sich der Gabelpunkt in eine bestimmte Richtung und um ein bestimmtes Maß verlagert.

Die Gabelarme können unterschiedlich lang sein. Obschon es möglich wäre, die Gabelarme gleich lang zu gestalten, beispielsweise durch unterschiedlich positionierte oder gekrümmte Gabelarmbögen, ergibt sich in besonders vorteilhafter Weise die gewünschte Unterstützung des gemeinsamen Gabelpunktes dann, wenn die Gabelarme unterschiedlich lang sind.

In einer weiteren Ausführungsform ist vorgesehen, dass die Gabelarme in einer Abwicklungsebene des Stents betrachtet einen ersten Längenabschnitt besitzen, in welchem die Gabelarme parallel zueinander verlaufen. Bereits diese parallel zueinander verlaufenden Längenabschnitte können unterschiedlich lang bemessen sein. Mit anderen Worten muss sich die unterschiedliche Länge der Gabelarme nicht zwangsläufig aus unterschiedlichen Krümmungen der Gabelarmbögen ergeben.

Bevorzugt besitzen die Konnektoren ausgehend von dem Bogen, an dem der Basisarm befestigt ist, hin zu den Bögen, an denen die Gabelarme befestigt sind, in einer Abwicklungsebene des Stents betrachtet einen S-förmig gekrümmten Verlauf. Das bedeutet, dass die Krümmungen der Gabelarme und die des Basisarms gegensätzlich sind. Der Gabelpunkt befindet sich außerhalb der Krümmungen etwa im mittleren Abstand zwischen den serpentinenartigen Elementen. Wenn jeder Arm, das heißt, die beiden Gabelarme und auch der Basisarm, einen Bogen besitzen, liegen die mittleren Bereiche des Y-förmigen Konnektors, das heißt auch der Bereich, in dem sich der Gabelpunkt befindet, etwa diagonal zur Längsachse des Stents.

Im Rahmen der Erfindung ist ferner vorgesehen, dass die in Radialrichtung gemessene Dicke der Arme eines Konnektors größer ist als die in Umfangsrichtung gemessene Breite der Arme. Das bedeutet, dass die Konnektoren vergleichsweise filigran gestaltet sind, aber in Umfangrichtung hinreichend biegesteif sind, so dass sie die gewünschte, ursprüngliche Krümmung auch in der expandierten Stellung beibehalten. Die serpentinenartigen Elemente, welche dafür zuständig sind, eine nach außen gerichtete Radialkraft aufzubringen, sind hingegen umgekehrt konfiguriert. Sie besitzen vorzugsweise eine in Radialrichtung gemessene Dicke, die geringer ist als die in Umfangsrichtung gemessene Breite. Das ist dadurch begründet, dass die serpentinenartigen Elemente insbesondere im Bereich der endseitigen Bögen eine hohe Widerstandskraft aufbringen müssen, um in der expandierten Position zu verbleiben. Der Bogen muss ein hinreichendes Widerstandsmoment besitzen, was nur durch entsprechende Querschnitte erreicht werden kann. Die Konnektoren, die aus fertigungstechnischen Gründen in derselben Abwicklungsebene des Stents liegen wie die serpentinenartigen Elemente, werden hingegen beim Expandieren des Stents insbesondere bei der Y-Variante tordiert. Um die Expansion des Stent nicht zu erschweren, darf das Widerstandsmoment gegen Torsion nicht zu groß sein. Daher sind die Konnektoren gewissermaßen schmaler als die serpentinenartigen Elemente, insbesondere als die Bögen. Die Bögen müssen hingegen die Torsionsmomente der Gabelarme und des Basisarms der Konnektoren aufnehmen, gleichzeitig eine von außen an den radial vorstehenden Konnektoren angreifende Kraft aufnehmen und zudem die serpentinenartigen Elemente in der expandierten Position halten. Daher ist der Bereich der Bögen am höchsten belastet und daher auch im Querschnitt am größten ausgeführt.

Es ist im Rahmen der Erfindung durchaus möglich, unterschiedliche Bauformen von Konnektoren miteinander zu kombinieren. Das heißt, es können in einem Längenabschnitt geteilte Konnektoren ausgebildet sein und in einem anderen Längenabschnitt ungeteilte Konnektoren.

Die Erfindung wird nachfolgend anhand der in den Zeichnungen schematisch dargestellten Ausführungsbeispiele näher erläutert.

Es zeigen:
- Figur 1: eine Ansicht auf eine Abwicklung der Mantelfläche eines Stents, der nicht ausgedehnt ist, in einer ersten Ausführungsform;
- Figur 2: eine vergrößerte Darstellung einzelner Konnektoren in der Ansicht wie in Figur 1;
- Figur 3: eine perspektivische Darstellung eines expandierten Stents in der Bauform der Figuren 1 und 2;
- Figur 4: eine vergrößerte Darstellung eines Teilbereichs der Figur 3;
- Figur 5: eine weitere vergrößerte Darstellung eines Teilbereichs der Figur 3;
- Figur 6: eine Ansicht auf eine Abwicklung eines Konnektors einer zweiten Ausführungsform;
- Figur 7: einen Stirnansicht des Stents der Figur 6 im expandierten Zustand;
- Figur 8: eine Stirnansicht durch des Stents der Figur 7 im nicht expandierten Zustand;
- Figur 9: eine Seitenansicht auf den Stent der zweiten Ausführungsform im expandierten Zustand;
- Figur 10: eine Seitenansicht auf den Stent der zweiten Ausführungsform im nicht expandierten Zustand und
- Figur 11: eine Ansicht auf eine Abwicklung der Mantelfläche eines Stents, der nicht ausgedehnt ist, in einer dritten Ausführungsform.

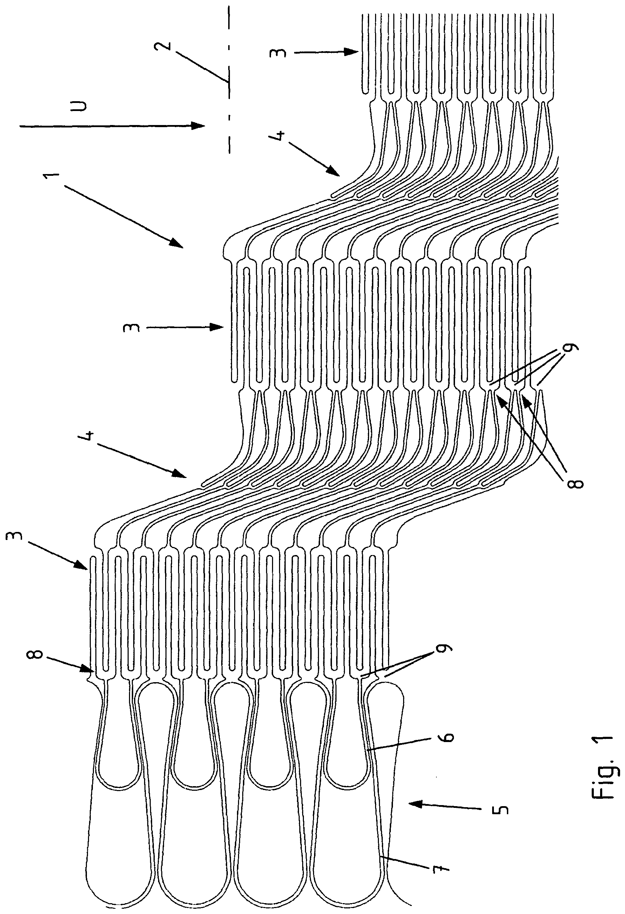

Figur 1 zeigt die Abwicklung eines Stents 1 in einer ersten Ausführungsform. Es handelt sich um das Schnittbild eines im Laserschnittverfahren hergestellten Metallbauteils. In der zylindrischen Form bildet diese Abwicklung einen rohrförmigen Körper, der um die Längsachse 2 geformt ist.

Der Stent 1 dieser Bauform besitzt im Wesentlichen drei unterschiedliche funktionale Abschnitte: Die massivsten Komponenten sind serpentinenartige, ringförmig ausdehnbare Elemente 3, wie sich in der Bildebene der Figur 1 von oben nach unten erstrecken. Innerhalb des rohrförmigen Körpers verlaufen die serpentinenartigen, ringförmig ausdehnbaren Elemente in Umfangsrichtung (Pfeil U). Diese Elemente 3 wiederholen sich in regelmäßigen axialen Abständen. Die einzelnen Elemente 3 sind über Konnektoren 4 miteinander verbunden. Während es sich bei dem serpentinenartigen, ringförmig ausdehnbaren Element 3 je Radialebene jeweils nur um ein einziges, umlaufendes Element 3 handelt, sind die in Axialrichtung einander benachbarten Elemente 3 über eine Vielzahl von Konnektoren 4 miteinander verbunden.

Der Stent 1 besitzt ferner einen Endabschnitt 5. Der Endabschnitt 5 besteht aus kürzeren, schlaufenartigen Bögen 6 sowie in Axialrichtung betrachtet längeren Bögen 7, welche die kürzeren Bögen 6 umgeben. Die kürzeren Bögen 6 sind jeweils mit einem Enden 8 des in der Bildebene linken, serpentinenartigen Elements 3 verbunden. Die Bögen 6, 7 des Endabschnitts 5 sind jeweils mit den endseitigen Bögen 9 des serpentinenartigen Elements verbunden. Dadurch verlaufen die größeren Bögen 7 im Endabschnitt 5 ähnlich wie die serpentinenartigen Elemente 3 mäanderförmig in Umfangsrichtung des Stents 1.

Der Stent 1 ist nicht in seiner vollen Länge dargestellt. Auch das zweite Ende des Stents 1 besitzt einen Endabschnitt 5 mit Bögen 6, 7.

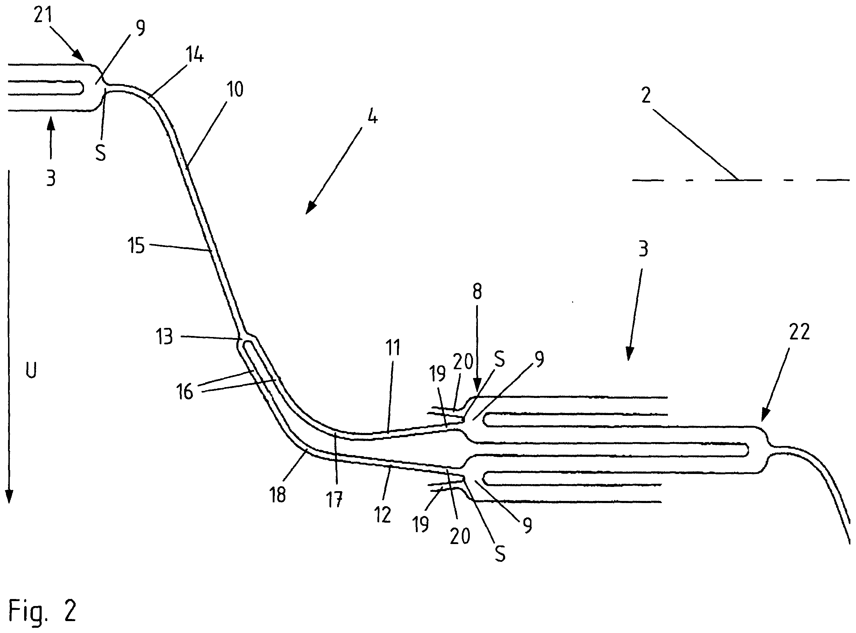

Figur 2 zeigt in vergrößerter Darstellung den näheren Aufbau der Konnektoren 4 und deren Verbindung mit den Elementen 3. Die Konnektoren 4 sind Y-förmig konfiguriert. Alle Konnektoren 4 sind identisch konfiguriert. Sie besitzen einen Basisarm 10 und jeweils zwei Gabelarme 11, 12. Etwa in der Mitte zwischen den beiden dargestellten, serpentinenartigen, ringförmig ausdehnbaren Elementen 3 befindet sich ein Gabelpunkt 13 des Konnektors 4.

Der Basisarm 10 ist im Scheitelpunkt S eines Bogens 9 am äußeren Umfang des Bogens 9 mit diesem Bogen 9 verbunden. Der Basisarm 10 zweigt gewissermaßen in Richtung der Längsachse 2 weisend von dem Bogen 9 ab. Der Basisarm 10 steht in unmittelbarer Nähe des Bogens 9 senkrecht auf dem Bogen 9. Es schließt sich im weiteren Verlauf des Basisarms 9 ein Basisarmbogen 14 an. In der Bildebene der Figur 2 knickt der Basisarm 10 dadurch nach unten in Richtung zum Gabelpunkt 13 ab. Die Verbindung zwischen dem Basisarmbogen 14 und dem Gabelpunkt 13 ist bei diesem Ausführungsbeispiel gerade und stellte den Hauptabschnitt 15 des Basisarms 10 dar. Der Hauptabschnitt 15 verläuft bei diesem Ausführungsbeispiel in einem eingeschlossenen Winkel von 70 Grad zur Längsachse 2. Erfindungsgemäß bewegt sich der eingeschlossene Winkel zwischen dem Hauptabschnitt 15 und der Längsachse 2 in einem Bereich von 50 bis 75 Grad.

Der Pfeil U kennzeichnet wieder die Umfangsrichtung des Stents. Es ist zu erkennen, dass durch die Abwinklung des Basisarms 10 der Gabelpunkt 13 in einem anderen Umfangsbereich liegt als der Bogen 9, an welchem der Basisarm 10 befestigt ist. Betrachtet man als Maßeinheit in Umfangsrichtung den Abstand der Scheitelpunkte S zweier Bögen 9, befindet sich der Gabelpunkt 13 in Umfangsrichtung U betrachtet etwa um 4 bis 5 Scheitelpunktabstände weiter versetzt als derjenige Bogen 9, an dem der Basisarm 10 befestigt ist.

Im zylindrischen Zustand, d. h. nicht in der Abwicklung des Zylindermantels des Stents, erstreckt sich der Konnektor 4 über einen Umfangsabschnitt des nicht expandierten Stents von 120° bis 240°. Beim Expandieren wird dieser Winkel kleiner und beträgt nur noch 30° bis 85°. Je stärker der Stent expandiert wird, desto kleiner wird der von den Konnektoren 4 überspannte Umfangsbereich und desto geringer wird die Überlappung der einzelnen Konnektoren 4. In der der dargestellten Abwicklung ist die Überlappung maximal.

Die beiden Gabelarme 11, 12 sind an einander benachbarten Bögen 9 des in der Bildebene rechten Elements 3 befestigt. Sie befindet sich etwa 5 bis 6 Scheitelpunktabstände in Umfangsrichtung U versetzt zu dem Bogen 9, an dem der Basisarm 10 befestigt ist.

Der Basisarm 10 besitzt eine konstante Breite. Die Gabelarme 11, 12 besitzen dieselbe Breite wie der Basisarm. Sie münden vom Gabelpunkt 13 ausgehend in einen ersten Längenabschnitt 16, in welchem die beiden Gabelarme 11, 12 parallel zueinander verlaufen. Der Längenabschnitt 16 des in der Bildebene oberen Gabelarms 11 ist etwas kürzer als der entsprechende Längenabschnitt 16 des unteren Gabelarms 12. An diese beiden Längenabschnitt, die zwar parallel zueinander verlaufen, jedoch nicht zu dem Hauptabschnitt 15 des Basisabschnitts fluchten, schließt sich jeweils ein Gabelarmbogen 17, 18 des jeweiligen Gabelarms 11, 12 an.

Der Längenabschnitt 16 ist um etwa 5 Grad gegenüber der Orientierung des Hauptabschnitts 15 abgewinkelt. Das heißt, die Längenabschnitte 16 der beiden Gabelarme 11, 12 liegen in einem Winkel von ca. 65 statt 70 Grad zur Längsachse 2. Die Abwinklung zwischen dem Basisarm 10 und den Gabelarmen 11, 12 kann 1 - 10° betragen.

Der Gabelarmbogen 17 des in der Bildebene oberen Gabelarms 11 erstreckt sich über einen größeren Winkelbereich als der andere Gabelarmbogen 18. Zwar ist der Krümmungsradius dieses ersten Gabelarmbogens 17 größer als der Krümmungsradius des anderen Gabelarmbogens, jedoch ist der obere Gabelabschnitt 17 trotz der geringeren Krümmung (Krümmung = Kehrwert des Krümmungsradius) insgesamt stärker gekrümmt als der untere Gabelabschnitt 18. Der obere Gabelarm 11 ist dadurch auch etwas kürzer als der andere Gabelarm 12, der sich in Umfangsrichtung U etwas weiter bis zum unteren der beiden Bögen 9 des in der Bildebene rechten Elements 3 erstrecken muss. An den Bögen 9 an den ersten Enden 8 sind jeweils zwei Gabelarmenden 19, 20 benachbarter Konnektoren 4 befestigt. Die Gabelarme 19, 20 sind benachbart dem Scheitelpunkt S des betreffenden Bogens 9 angeordnet und zweigen V-förmig benachbart vom Scheitelpunkt S des Bogens 9 ab. Bedingt durch die V-Form sind die Gabelarme 11, 12 im Bereich ihrer Gabelarme 19, 20 in größerem Abstand zueinander angeordnet als im Bereich der Gabelbögen 17, 18 bzw. als in den parallelen Längenabschnitten 16. Die Gabelarme 11, 12 sind dadurch gewissermaßen V-förmig aufgespreizt.

Insgesamt verläuft jeder einzelne Konnektor 4 S-förmig zwischen ersten Enden 21 und zweiten Enden 8 einander benachbarter, serpentinenartiger, ringförmig ausdehnbarer Elemente 3.

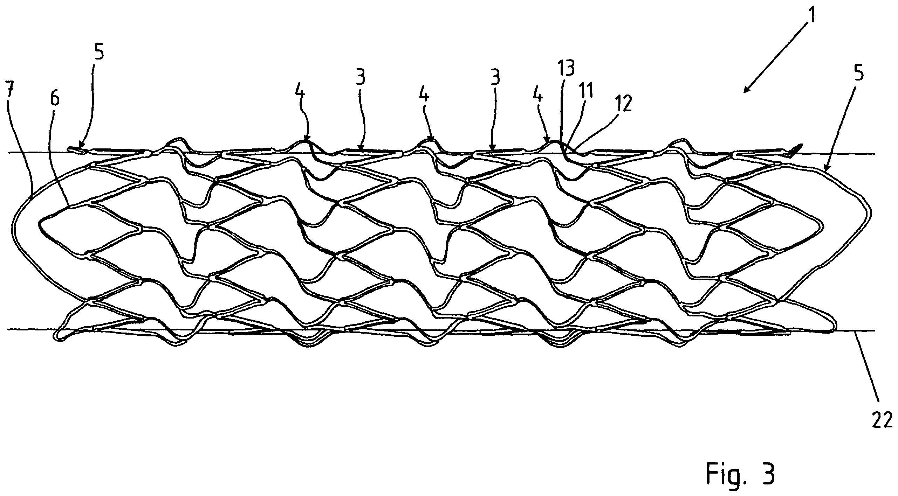

Während die Figuren 1 und 2 den Stent 1 in einer Abwicklung, das heißt in noch nicht rohrförmigen Zustand und im nicht expandierten Zustand, zeigen, verdeutlichen die Figuren 3 bis 5, wie der Stents 1 im expandierten Zustand aussieht.

Figur 3 zeigt den Stent 1 der Figuren 1 und 2. Der Stent 1 befindet sich zur besseren Veranschaulichungen auf einem Zylinder 22. Der Zylinder 22 soll den Zustand des Stents 1 während des Expandierens verdeutlichen. Nach dem Expandieren wird der Zylinder 22 wieder entfernt, so dass der Stent 1 in der Körperhöhle bleibt und diese offen hält.

Der Stent 1 besitzt wiederum die mehreren, serpentinenartigen, ringförmigen und nunmehr ausgedehnten Elemente 3 und die zwischen den Elementen 3 angeordneten, Y-förmig konfigurierten Konnektoren 4. Darüber hinaus zeigt Figur 3 Endabschnitte 5 mit den inneren und äußeren Bögen 6, 7 im expandierten Zustand.

Es ist zu erkennen, dass die Konnektoren 4 deutlich nach oben und unten, d. h. in Radialrichtung, über den Zylinder 22 vorstehen. Sie befinden sich in der expandierten Position, also nicht mehr vollständig in derselben zylindrischen Mantelfläche wie die serpentinenartigen Elemente 3, die sich insgesamt eng an den Zylinder 23 anschmiegen und gewissermaßen das Gerüst für die rohrförmige Struktur des Stents 1 bilden. Die Konnektoren 4 sind im Bereich ihrer Gabelarme 11, 12 stark gespreizt. Der Gabelpunkt 13 ist nach radial außen verlagert und hebt sich von dem Zylindermantel 22 ab.

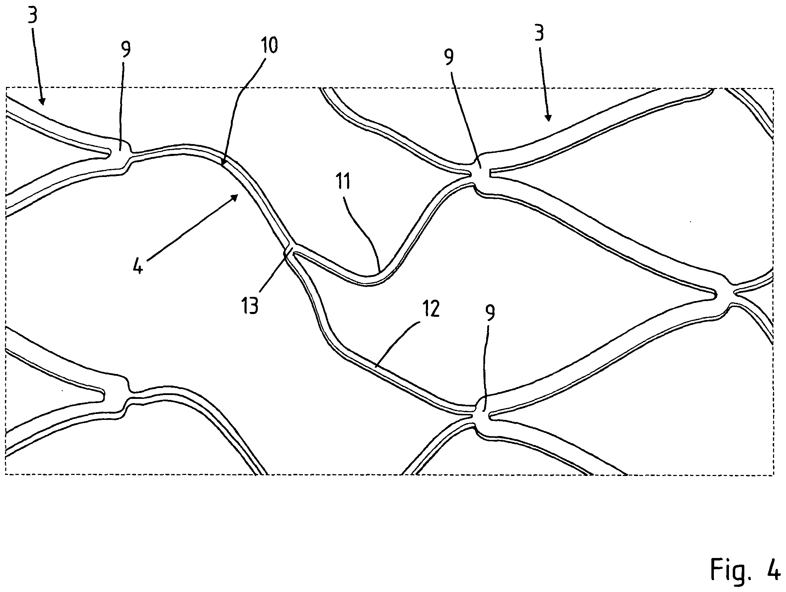

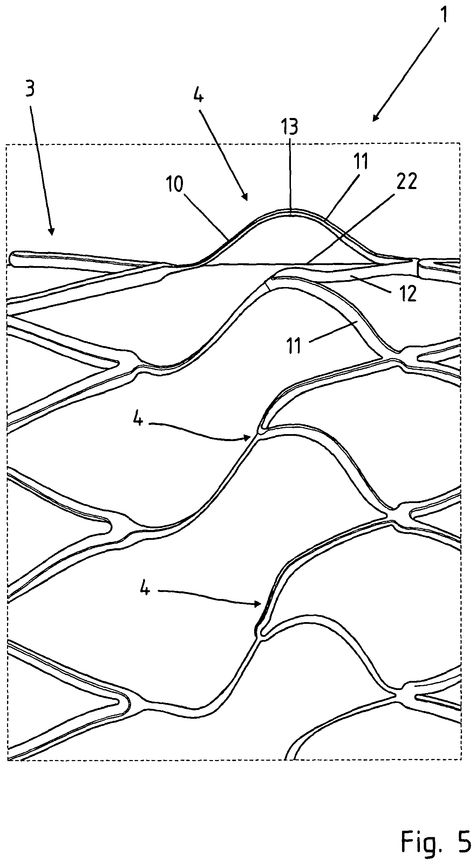

Die Darstellung der Figur 4 zeigt in einer Vergrößerung, dass die einzelnen Gabelarme 11, 12 vergleichsweise stark verformt werden. Es wird von den beiden Gabelarmen 11, 12 durch das Spreizen der serpentinenartigen, ringförmig ausdehnbaren Elemente 3 ein relativ großer Abstand zwischen den Bögen 9 der Elemente 3 überbrückt. Dadurch verwinden sich die Gabelarme 11, 12 in entgegengesetzte Richtungen und üben jeweils in entgegengesetzte Richtungen wirkende Drehmomente auf den Gabelpunkt 13 des Konnektors 4 aus. Gleichzeitig greift der Basisarm 10 am Gabelpunkt 13 an und stützt diesen. Durch die Drehmomente, welche die Gabelarme 11, 12 ausüben, wird der Basisarm 10 gegenüber dem Bogen 9, an dem der Basisarm 10 befestigt ist, verschwenkt. Der mittlere Bereich des Konnektors 4 wird aus der Mantelfläche, die von den serpentinenartigen Elementen 3 aufgespannt wird, abgehoben. Das wird insbesondere in Figur 5 deutlich. Der in der Bildebene der Figur 5 oberste Konnektor 4 ist in seinem mittleren Bereich deutlich von dem Zylinder 22 abgehoben. Der Konnektor 4 dient als zusätzliche Verankerung des Stents 1 in dem umliegenden Gewebe (nicht näher dargestellt). In gleicher Weise verlaufen auch die übrigen Konnektoren 4, das heißt, die Gabelpunkte 13 zwischen den Gabelarmen 11, 12 und dem Basisarm 10 sind deutlich von dem Zylinder 22 abgehoben, so dass sich insgesamt eine umlaufende, wulstartige Erhöhung der dargestellten Gitterstruktur des Stents 1 ergibt.

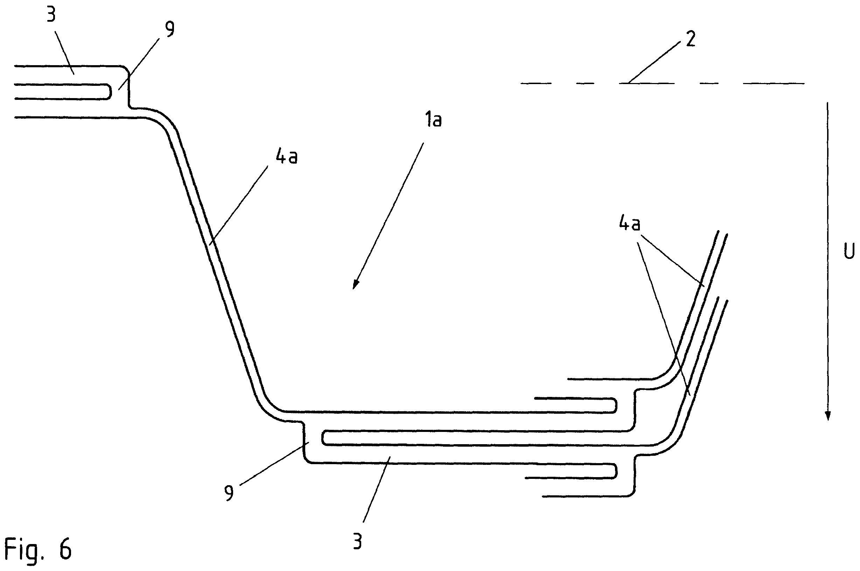

Die Figur 6 zeigt einen Teilbereich einer Abwicklung einer zweiten Ausführungsform eines Stents 1 a. Dieser Stent 1 a besitzt keine geteilten Konnektoren, sondern ungeteilte Konnektoren 4a. Sie verlaufen ebenfalls S-förmig.

Die serpentinenartigen Elemente 3 sind im Wesentlichen identisch. Die Konnektoren 4a sind allerdings nicht im Scheitelpunkt der Bögen 9 angeordnet, sondern setzen an einer Ecke der Bögen 9 an und bilden daher gewissermaßen eine Verlängerung einer Längsseite eines serpentinenartigen Elements 3. Die Konnektoren 4a sind in ihrem weiteren Verlauf im Wesentlichen gerade. Sie stehen im Winkel zur Umfangsrichtung U und auch zur Längsachse 2.

Ein weiterer Unterschied zur ersten Ausführungsform ist, dass die Konnektoren 4a in der Bildebene rechts nicht nach unten weisen, sondern nach oben. Das heißt, dass die Konnektoren 4a gegensätzlich oder auch gegensinnig orientiert sind.

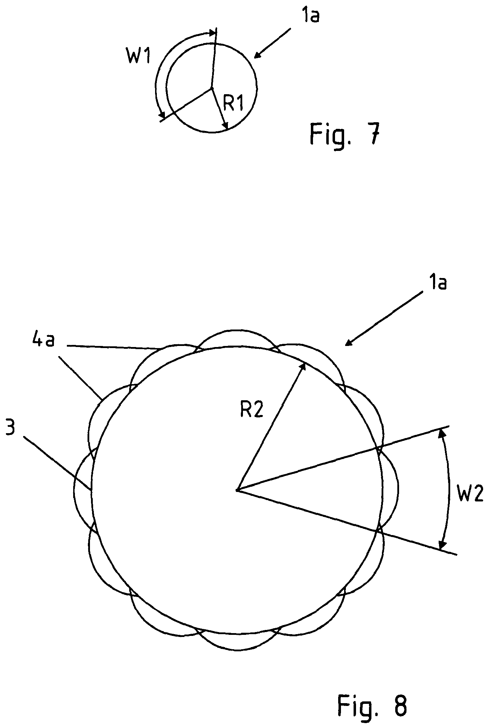

Figur 7 zeigt rein schematisch den Stent 1 a im nicht expandierten Zustand im Querschnitt. Der Radius R1 beträgt z.B. 2,5 mm. Die Konnektoren 4a erstrecken sich über einen Winkel W1 von 120° bis 130°. Ihre Krümmung K1 ist der Kehrwert des Radius R1 (K1 = 1/R1)

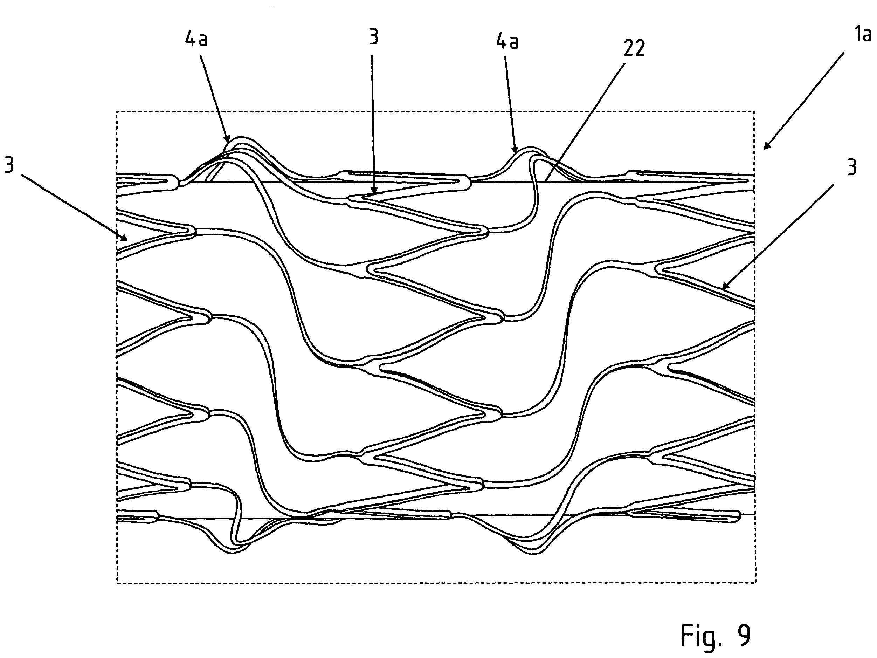

Figur 8 zeigt den Stent 1a der Figur 7 im expandierten Zustand, ebenfalls rein schematisch. Der Radius hat sich etwas um den Faktor 4 vergrößert und wird nun als R2 bezeichnet. Die Krümmung K2 des Zylindermantels hat sich folglich etwa um den Faktor 4 verkleinert (K2 = 1/R2). Die Konnektoren 4a haben allerdings auch im expandierten Zustand des Stents 1a ihre ursprüngliche Krümmung K1 = 1/R1 behalten. Die Konnektoren 4a treten dadurch deutlich als einzelne Bögen über den dargestellten Kreis, d.h. den von den serpentinenartigen Elementen 3 aufgespannten Zylindermantel, vor. Dabei überlappen sich die Konnektoren 4a in Umfangsrichtung ein wenig. Es entsteht ein umlaufender Wulst. Jeder Konnektor 4a überspannt dabei einen viel kleineren Winkel W2, der bei diesem Ausführungsbeispiel etwa 30° beträgt, so dass 12 Konnektoren 4a erforderlich sind, um den gesamten Wulst zu bilden. Figur 9 zeigt in einer schematischen, dreidimensionalen Darstellung den Wulst, wie er von den Konnektoren 4a gebildet wird. Zur besseren Veranschaulichung ist der rückwärtige Teil des Stents 1a von einem Zylinder 22, der sich innerhalb des Stents 1 a befindet, bedeckt. Auch diese Ausführungsform kann mit Endabschnitten 5 wie in Figur 3 versehen sein. Der Unterschied zwischen den Varianten ist lediglich in der Gestaltung der Konnektoren 4a zu sehen.

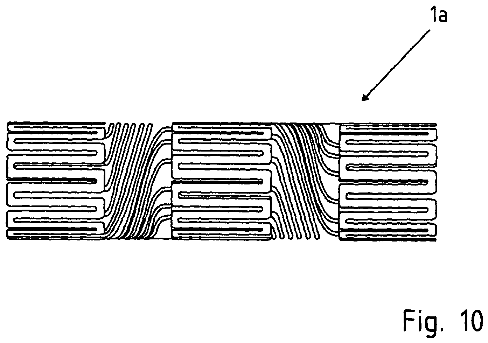

Die Figur 10 zeigt noch einmal den Stent 1 a mit einteiligen Konnektoren 4a im nicht expandierten Zustand in der Seitenansicht.

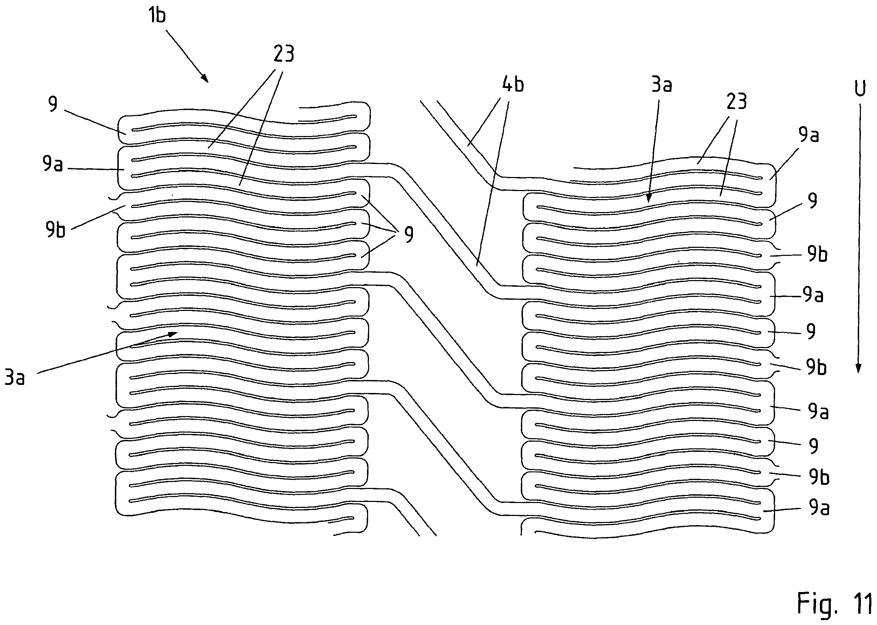

Figur 11 zeigt die Abwicklung eines Stents 1 b. Die Konnektor 4b sind wiederum ungeteilt. Der Unterschied gegenüber der Ausführungsform der Figur 6 ist, dass die Konnektoren 4b nicht an Außenseiten der Bögen 9 befestigt sind, sondern an Innenseiten der Bögen 9a. Dazu erstrecken sich die Konnektoren 4b bis in den von den serpentinenartigen Elementen 3a überdeckten Bereich hinein. Sie verlaufen innerhalb der serpentinenartigen Elemente 3a zwischen und parallel zu den Schenkeln 23 der serpentinenartigen Elemente 3a. Als Schenkel 23 werden die Bereiche zwischen den Bögen 9, 9a der serpentinenartigen Elemente 3a verstanden. Die Schenkel 23 sind bei diesem Ausführungsbeispiel leicht gewellt. Der Verlauf der Konnektoren 4b ist in gleicher Weise gewellt, so dass die Konnektoren 4b und Schenkel 23 parallel verlaufen.

Ein weiterer Unterschied ist, dass die Konnektoren 4b nicht an jedem möglichen Bogen 9, 9a, 9b befestigt sind, sondern nur innenseitig an jedem dritten Bogen 9a. Dafür sind Bögen 9a in Umfangsrichtung U betrachtet etwas breiter.

Ein weiterer Unterschied ist, dass dieses Design der Konnektoren 4b sich nicht mehrfach über die Länge des Stents 1 b wiederholt. Es ist zu erkennen, dass es einen weiteren Typus von Bogen 9b gibt, und zwar auf der den Konnektoren 4b angewandten Seite der serpentinenartigen Elemente 3a. Dieser Typ Bogen 9b ist außenseitig mit einem nicht näher dargestellten Arm eines weiteren Konnektors verbunden. Es kann sich um einen geteilten oder ungeteilten Konnektor handeln. In der Bildebene links verhält es sich genauso. Das heißt es gibt eine wiederkehrende Abfolge von drei unterschiedlichen Bögen 9, 9a, 9b, nämlich dem einfachen Bogen 9 ohne angeschlossenen Konnektor, dann einen Bogen 9a mit sich innenseitig anschließenden Konnektor 4b und danach einen Bogen 9b mit sich außenseitig anschließenden Konnektor, wobei der daran angeschlossene Konnektor z.B. das Design wie in Figur 9 haben kann.

### Bezugszeichen:

- 1 -: Stent
- 1a -: Stent
- 1b -: Stent
- 2 -: Längsachse
- 3 -: serpentinenartige Elemente
- 3a -: serpentinenartige Elemente
- 4 -: Konnektoren
- 4a -: Konnektoren
- 4b -: Konnektoren
- 5 -: Endabschnitt
- 6 -: Bogen
- 7 -: Bogen
- 8 -: Ende
- 9 -: Bogen
- 9a -: Bogen
- 9b -: Bogen
- 9c -: Bogen
- 10 -: Basispunkt
- 11 -: Gabelarm
- 12 -: Gabelarm
- 13 -: Gabelpunkt
- 14 -: Basisarmbogen
- 15 -: Hauptabschnitt
- 16 -: Längenabschnitt
- 17 -: Gabelarmbogen
- 18 -: Gabelarmbogen
- 19 -: Gabelarmende v. 11
- 20 -: Gabelarmende v. 12
- 21 -: zweites Ende
- 22 -: Zylinder
- 23 -: Schenkel
- K1 -: Krümmung
- K2 -: Krümmung
- R1 -: Radius
- R2 -: Radius
- S -: Scheitelpunkt
- U -: Umfangsrichtung
- W1 -: Winkel
- W2 -: Winkel

## Patentansprüche

1. Stent in Form eines im allgemeinen rohrförmigen Körpers mit mehreren serpentinenartigen, ringförmig ausdehnbaren Elementen (3, 3a), wobei die serpentinenartigen Elemente (3, 3a) Bögen (9, 9a, 9b) an ihren in Axialrichtung des Stents (1) weisenden ersten und zweiten Enden (8, 19, 21) besitzen, wobei in Axialrichtung aufeinander folgende, serpentinenartige, ringförmig ausdehnbare Elemente (3, 3a) über Konnektoren (4, 4a, 4b) miteinander verbunden sind, die an den Enden (8, 21) der benachbarten Elemente (3, 3a) angeordnet sind, wobei die Konnektoren (4, 4a) sich in einem nicht expandierten Zustand des Stents (1, 1 a, 1 b) in Umfangsrichtung des Stents (1, 1 a, 1 b) erstrecken und eine Krümmung (K1) in Umfangsrichtung (U) besitzen, wobei die Krümmung (K1) im expandierten Zustand des Stents (1, 1 a, 1 b) in Umfangrichtung (U) so groß ist, dass die Konnektoren (4, 4a, 4b) nach radial außen über den Umfang der serpentinenartigen Elemente (3, 3a) vorstehen, wobei die Konnektoren (4) Y-förmig konfiguriert sind, wobei ein Basisarm (10) des Konnektors (4) mit einem Bogen (9) am Ende (21) des ersten Elements (3) verbunden ist und die beiden Gabelarme (11, 12) des Y-förmigen Konnektors (4) jeweils mit einem von zwei benachbarten Bögen (9) am Ende (8) des anderen Elements (3) verbunden sind.

2. Stent nach Anspruch 1, **dadurch gekennzeichnet, dass** sich die Konnektoren (4, 4a, 4b) im nicht expandierten Zustand des Stents (1, 1a, 1b) über einen Winkel (W1) in einem Bereich von 120° bis 240 erstrecken.

3. Stent nach Anspruch 1 oder 2, **dadurch gekennzeichnet, dass** sich ein Konnektor (4, 4a, 4b) im expandierten Zustand des Stents (1, 1 a, 1 b) über einen Winkel (W2) in einem Bereich von 30° bis 85° erstrecken.

4. Stent nach einem der Ansprüche 1 bis 3, **dadurch gekennzeichnet, dass** in Längsrichtung (2) aufeinander folgende Konnektoren (4, 4a, 4b) gegensinnig orientiert verlaufen.

5. Stent nach Anspruch 1, **dadurch gekennzeichnet, dass** alle Bögen (9) eines serpentinenartigen Elements (3) über die Konnektoren (4) mit den Bögen (9) eines benachbarten, serpentinenartigen Elements (3) verbunden sind.

6. Stent nach einem der Ansprüche 1 bis 5, **dadurch gekennzeichnet, dass** in einer Abwicklungsebene des Stents betrachtet der Basisarm (10) gekrümmt ist, so dass der mit dem Basisarm (10) verbundene Bogen (9) des einen Elements (3) in einem anderen Umfangsabschnitt als die Bögen (9) liegt, mit welchen die Gabelarme (11, 12) verbunden sind.

7. Stent nach einem der Ansprüche 1 bis 6, **dadurch gekennzeichnet, dass** in einer Abwicklungsebene des Stents betrachtet die Gabelarme (11, 12) gekrümmt sind, so dass der Gabelpunkt (13), an dem der Basisarm (10) mit den beiden Gabelarmen (11, 12) verbunden ist, in einem anderen Umfangsabschnitt liegt als die Bögen (9), mit welchen die Gabelarme (11, 12) verbunden sind.

8. Stent nach Anspruch 7, **dadurch gekennzeichnet, dass** die Gabelarme (11, 12) zur Krümmung jeweils einen Gabelarmbogen (17, 18) besitzen, wobei die Gabelarmbögen (17, 18) in ihrem Krümmungsradius (R1, R2) in einer Abwicklungsebene des Stents betrachtet voneinander abweichen.

9. Stent nach einem der Ansprüche 1 bis 8, **dadurch gekennzeichnet, dass** die Gabelarme (11, 12) eines Konnektors (4) unterschiedlich lang sind.

10. Stent nach einem der Ansprüche 1 bis 9, **dadurch gekennzeichnet, dass** die Gabelarme (11, 12) einen ersten Längenabschnitt (16) besitzen, wobei diese ersten Längenabschnitte (16) in einer Abwicklungsebene des Stents betrachtet parallel zueinander verlaufen und unterschiedlich lang sind.

11. Stent nach einem der Ansprüche 1 bis 9, **dadurch gekennzeichnet, dass** die Konnektoren (4) von dem Bogen (9), an dem der Basisarm (10) befestigt ist, hin zu den Bögen (9), an denen die Gabelarme (11, 12) befestigt sind, in einer Abwicklungsebene des Stents betrachtet einen S-förmig gekrümmten Verlauf haben.

12. Stent nach einem der Ansprüche 1 bis 11, **dadurch gekennzeichnet, dass** eine in Radialrichtung gemessene Dicke der besagten Arme (10, 11, 12) eines Konnektors (4) größer ist als die im Umfangsrichtung (U) gemessene Breite der besagten Arme (10, 11, 12).

## Claims

1. Stent in the form of a generally tubular member having a plurality of serpentine-like, annularly expandable elements (3, 3a), wherein the serpentine-like elements (3, 3a) have curves (9, 9a, 9b) at the first and second ends (8, 19, 21) thereof facing in the axial direction of the stent (1), wherein serpentine-like elements (3, 3a) which are successive in the axial direction and which can be expanded in an annular manner are connected to each other by means of connectors (4, 4a, 4b) which are arranged at the ends (8, 21) of the adjacent elements (3, 3a), wherein the connectors (4, 4a) in a non-expanded state of the stent (1, 1a, 1b) extend in the peripheral direction of the stent (1, 1a, 1b) and have a curvature (K1) in the peripheral direction (U), wherein the curvature (K1) in the expanded state of the stent (1, 1a, 1b) is so large in the peripheral direction (U) that the connectors (4, 4a, 4b) protrude radially outwards over the periphery of the serpentine-like elements (3,3a), wherein the connectors (4) are configured in a Y-shaped manner, wherein a base arm (10) of the connector (4) is connected to a curve (9) at the end (21) of the first element (3) and the two fork arms (11, 12) of the Y-shaped connector (4) are each connected to one of two adjacent curves (9) at the end (8) of the other element (3).

2. Stent according to claim 1, **characterised in that** the connectors (4, 4a, 4b) in the non-expanded state of the stent (1, 1a, 1b) extend over an angle (W1) in a range from 120° to 240°.

3. Stent according to claim 1 or 2, **characterised in that** a connector (4, 4a, 4b) in the expanded state of the stent (1, 1a, 1b) extends over an angle (W2) in a range from 30° to 85°.

4. Stent according to any one of claims 1 to 3, **characterised in that** connectors (4, 4a, 4b) which are successive in the longitudinal direction (2) extend so as to be orientated in opposite directions.

5. Stent according to claim 1, **characterised in that** all the curves (9) of a serpentine-like element (3) are connected by means of the connectors (4) to the curves (9) of an adjacent serpentine-like element (3).

6. Stent according to any one of claims 1 to 5, **characterised in that**, when viewed in a development plane of the stent, the base arm (10) is curved so that the curve (9) of one element (3), which curve is connected to the base arm (10), is located in a different peripheral portion from the curves (9) to which the fork arms (11, 12) are connected.

7. Stent according to any one of claims 1 to 6, **characterised in that**, when viewed in a development plane of the stent, the fork arms (11, 12) are curved so that the fork point (13) at which the base arm (10) is connected to the two fork arms (11, 12) is located in a different peripheral portion to the curves (9) to which the fork arms (11, 12) are connected.

8. Stent according to claim 7, **characterised in that** the fork arms (11, 12) for curvature each have a fork arm curve (17, 18), wherein the fork arm curves (17, 18) differ from each other in terms of their radius of curvature (R1, R2) when viewed in a development plane of the stent.

9. Stent according to any one of claims 1 to 8, **characterised in that** the fork arms (11, 12) of a connector (4) are of different lengths.

10. Stent according to any one of claims 1 to 9, **characterised in that** the fork arms (11, 12) have a first longitudinal portion (16), wherein these first longitudinal portions (16), when viewed in a development plane of the stent, extend parallel with each other and have different lengths.

11. Stent according to any one of claims 1 to 9, **characterised in that** the connectors (4) from the curve (9), to which the base arm (10) is secured, to the curves (9), to which the fork arms (11, 12) are secured, when viewed in a development plane of the stent, have an S-shaped curved path.

12. Stent according to any one of claims 1 to 11, **characterised in that** a thickness of the said arms (10, 11, 12) of a connector (4) as measured in the radial direction is greater than the width of the said arms (10, 11, 12) as measured in the peripheral direction (U).

## Revendications

1. Endoprothèse sous la forme d'un corps généralement de forme tubulaire avec plusieurs éléments (3, 3a) en serpentin, extensibles de manière à présenter une forme annulaire, dans laquelle les éléments (3, 3a) en serpentin possèdent des arcs (9, 9a, 9b) au niveau de leurs première et deuxième extrémités (8, 19, 21) pointant dans la direction axiale de l'endoprothèse (1), dans laquelle des éléments (3, 3a) se suivant les uns les autres dans la direction axiale, en serpentin, extensibles de manière à présenter une forme annulaire sont reliés les uns aux autres par l'intermédiaire de connecteurs (4, 4a, 4b), qui sont disposés au niveau des extrémités (8, 21) des éléments (3, 3a) adjacents, dans laquelle les connecteurs (4, 4a) s'étendent, dans un état non expansé de l'endoprothèse (1, 1a, 1b), dans la direction périphérique de l'endoprothèse (1, 1a, 1b) et possèdent une courbure (K1) dans la direction périphérique (U), dans laquelle la courbure (K1) est, dans l'état expansé de l'endoprothèse (1, 1a, 1b), si grande dans la direction périphérique (U) que les connecteurs (4, 4a, 4b) font saillie vers l'extérieur radialement de la périphérie des éléments (3, 3a) en serpentin, dans laquelle les connecteurs (4) sont configurés de manière à présenter une forme de Y, dans laquelle un bras de base (10) du connecteur (4) est relié à un arc (9) au niveau de l'extrémité (21) du premier élément (3) et les deux bras de bifurcation (11, 12) du connecteur (4) en forme de Y sont reliés respectivement à l'un de deux arcs (9) adjacents au niveau de l'extrémité (8) de l'autre élément (3).

2. Endoprothèse selon la revendication 1, **caractérisée en ce que** les connecteurs (4, 4a, 4b) s'étendent, dans l'état non expansé de l'endoprothèse (1, 1a, 1b), sur un angle (W1) situé dans une plage allant de 120° à 240°.

3. Endoprothèse selon la revendication 1 ou 2, **caractérisée en ce qu'**un connecteur (4, 4a, 4b) s'étend, dans l'état expansé de l'endoprothèse (1, 1a, 1b), sur un angle (W2) situé dans une plage allant de 30° à 85°.

4. Endoprothèse selon l'une quelconque des revendications 1 à 3, **caractérisée en ce que** des connecteurs (4, 4a, 4b) se suivant dans la direction longitudinale (2) s'étendent en étant orientés en sens inverse.

5. Endoprothèse selon la revendication 1, **caractérisée en ce que** tous les arcs (9) d'un élément (3) en serpentin sont reliés par l'intermédiaire des connecteurs (4) aux arcs (9) d'un élément (3) en serpentin adjacent.

6. Endoprothèse selon l'une quelconque des revendications 1 à 5, **caractérisée en ce que** vu dans un plan de projection développée de l'endoprothèse, le bras de base (10) est courbé de sorte que l'arc (9) relié au bras de base (10) de l'un des éléments (3) se trouve dans un autre tronçon périphérique que les arcs (9), auxquels les bras de bifurcation (11, 12) sont reliés.

7. Endoprothèse selon l'une quelconque des revendications 1 à 6, **caractérisée en ce que** vu dans un plan de projection développée de l'endoprothèse, les bras de bifurcation (11, 12) sont courbés, de sorte que le point de bifurcation (13), au niveau duquel le bras de base (10) est relié aux deux bras de bifurcation (11, 12), se trouve dans un autre tronçon périphérique que les arcs (9), auxquels les bras de bifurcation (11, 12) sont reliés.

8. Endoprothèse selon la revendication 7, **caractérisée en ce que** les bras de bifurcation (11, 12) possèdent aux fins de la courbure respectivement un arc de bras de bifurcation (17, 18), dans laquelle les arcs de bras de bifurcation (17, 18) divergent les uns des autres eu égard à leur rayon de courbure (R1, R2) vus dans un plan de projection développée de l'endoprothèse.

9. Endoprothèse selon l'une quelconque des revendications 1 à 8, **caractérisée en ce que** les bras de bifurcation (11, 12) d'un connecteur (4) présentent une longueur différente.

10. Endoprothèse selon l'une quelconque des revendications 1 à 9, **caractérisée en ce que** les bras de bifurcation (11, 12) possèdent un premier tronçon de longueur (16), dans laquelle lesdits premiers tronçons de longueur (16) s'étendent, vus dans un plan de projection développée de l'endoprothèse, de manière parallèle les uns par rapport aux autres et présentent une longueur différente.

11. Endoprothèse selon l'une quelconque des revendications 1 à 9, **caractérisée en ce que** les connecteurs (4) ont, depuis l'arc (9), au niveau duquel le bras de base (10) est fixé, en direction des arcs (9), au niveau desquels les bras de bifurcation (11, 12) sont fixés, vus dans un plan de projection développée de l'endoprothèse, un profil courbé en forme de S.

12. Endoprothèse selon l'une quelconque des revendications 1 à 11, **caractérisée en ce qu'**une épaisseur mesurée dans la direction radiale desdits bras (10, 11, 12) d'un connecteur (4) est plus grande que la largeur mesurée dans la direction périphérique (U) desdits bras (10, 11, 12).
